# EUROPEAN PATENT APPLICATION

(11) **EP 4 108 302 A1**
(43) Date of publication of application: **28.12.2022**
(21) Application number: 20920167.2
(22) Date of filing: 11.12.2020
(51) Int. Cl.: A63B 26/00, F24F 1/0076, F24F 1/0353, A61L 9/22

(54) **EXERCISE SYSTEM, EXERCISE DEVICE, AND EXERCISE METHOD**

(30) Priority: 20.02.2020 JP 2020027223
(71) Applicant: Sharp Kabushiki Kaisha, Sakai-shi Osaka 590-8522 (JP)
(72) Inventor: MATSUMOTO Masaru, Sakai City, Osaka 590-8522 (JP); FUNAMORI Hirokazu, Sakai City, Osaka 590-8522 (JP)
(74) Representative: Treeby, Philip David William
(86) International application number: PCT/JP2020/046339
(87) International publication number: WO 2021/166390

(57) **Abstract**

An exercise system (1) includes a load applying apparatus (10) and an ion delivery apparatus (20). The load applying apparatus (10) is disposed in a room (30) and configured to apply a load to a body of a user H. The ion delivery apparatus (20) is disposed in the room (30) and configured to deliver ions. The ion delivery apparatus (20) delivers ions toward a side of the load applying apparatus (10). The ion delivery apparatus (20) delivers ions in a direction of the user H exercising using the load applying apparatus (10). The ion delivery apparatus (10) delivers ions to a specific portion of the user H. The specific portion refers to a portion where skin of the user H is exposed. A plurality of the ion delivery apparatuses (20) is provided. The plurality of the ion delivery apparatuses (20) is disposed at different positions.

## Description

### Technical Field

The present invention relates to an exercise system, an exercise apparatus, and an exercise method.

### Background Art

The exercise apparatus described in PTL 1 includes a weight, a handle, and a wire. The weight has a predetermined weight. The handle is connected to the weight via the wire. The wire connects the weight and the handle. The weight is pulled up in response to a force exerted by a user via the handle. The pulled up weight reaches a predetermined position. The weight is then moved from the predetermined position toward the initial position.

### Citation List

### Patent Literature

PTL 1: JP 2012-170754A

### Summary of Invention

### Technical Problem

However, the exercise apparatus described in PTL 1 cannot improve the effects of the exercise depending on the user's psychological state.

In light of the problem described above, the present invention is directed at providing an exercise system, an exercise apparatus, and an exercise method capable of improving the effects of exercise.

### Solution to Problem

According to an aspect of the present invention, an exercise system includes a load applying apparatus and an ion delivery apparatus. The load applying apparatus is disposed in a room and configured to apply a load to a body of a user. The ion delivery apparatus is disposed in the room and configured to deliver ions.

According to an aspect of the present invention, an exercise apparatus includes a load applying unit and an ion delivery unit. The load applying unit is disposed in a room and configured to apply a load to a body of a user. The ion delivery unit is disposed in the room and configured to deliver ions.

According to an aspect of the present invention, an exercise method includes applying and delivering. In the applying, a load is applied to a body of a user by a load applying unit disposed in a room. In the delivering, ions are delivered by an ion delivery unit disposed in the room.

### Advantage Effects of Invention

According to an exercise system, an exercise apparatus, and an exercise method of the present invention, the effects of exercise can be improved.

### Brief Description of Drawings

FIG. 1 is a diagram illustrating an exercise system according to an embodiment of the present invention.
FIG. 2 is another diagram illustrating an exercise system according to an embodiment of the present invention.
FIG. 3 is a diagram illustrating an ion delivery apparatus according to an exercise system of a present embodiment.
FIG. 4 is a diagram illustrating the processing executed by the exercise system according to the present embodiment.
FIG. 5 is a diagram illustrating the experimental process of experiments using the exercise system according to the present embodiment.
FIG. 6 is a graph showing the average values of the amount of change in the activity level in an example of the present invention and a comparative example.
FIG. 7 is a graph showing the average values of the amount of change in the arousal level in the example of the present invention and the comparative example.
FIG. 8 is a graph showing the average values of the amount of change in the comfort level in the example of the present invention and the comparative example.
FIG. 9 is another graph showing the average values of the amount of change in the activity level in the example of the present invention and the comparative example.
FIG. 10 is a graph showing the average values of the amount of change in the fatigue level in the example of the present invention and the comparative example.
FIG. 11 is a graph showing the average values of the rate of perceived exertion in the example of the present invention and the comparative example.
FIG. 12 is a graph showing the average values of the load amount of exercise of a fourth process in the example of the present invention and comparative example.
FIG. 13 is a graph showing the average values of the load amount of exercise of a sixth process in the example of the present invention and comparative example.

### Description of Embodiments

Embodiments of the present invention will be described hereinafter with reference to the drawings. In the drawings, the same or equivalent components are denoted by the same reference signs, and description thereof will not be repeated.

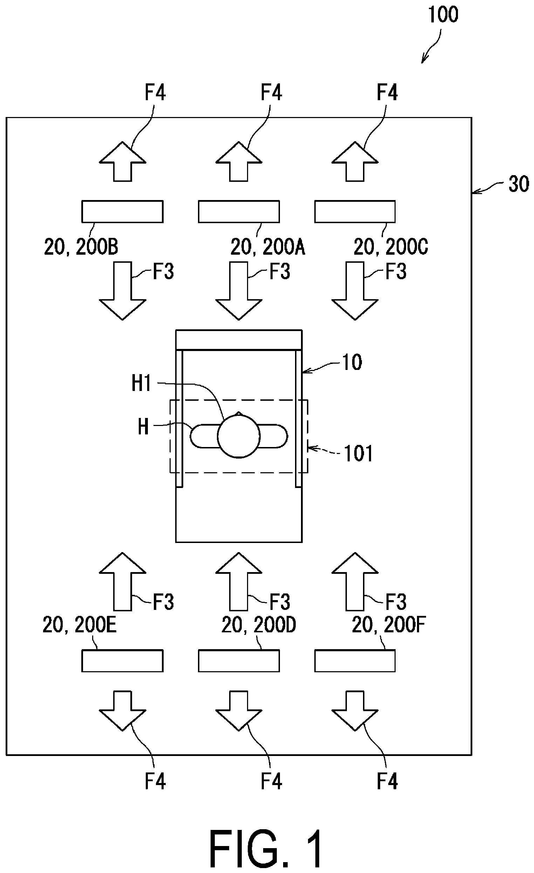

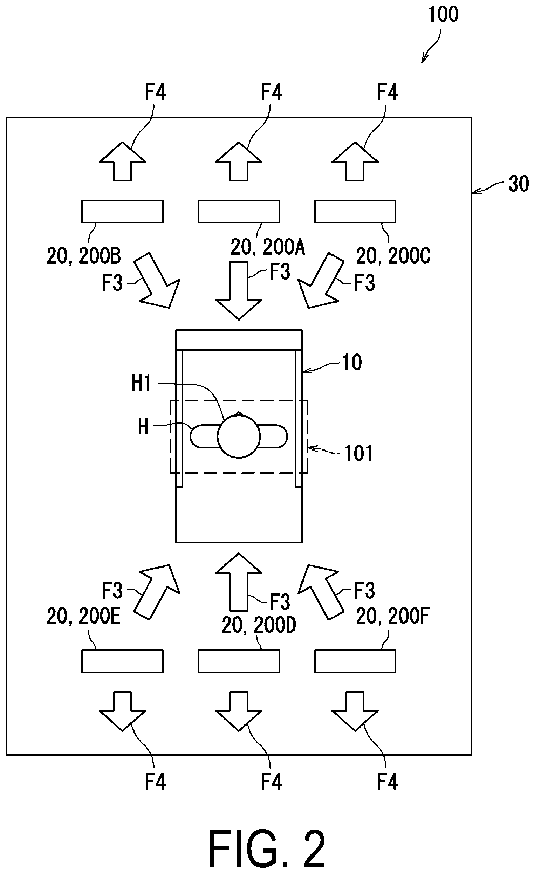

First, with reference to FIGS. 1 and 2, an exercise system 1 according to an embodiment of the present invention will be described. FIG. 1 is a diagram illustrating the exercise system 1 according to an embodiment of the present invention. FIG. 2 is another diagram illustrating the exercise system 1 according to an embodiment of the present invention. The exercise system 1 makes a user H exercise. Exercise includes aerobic exercise and anaerobic exercise. The aerobic exercise refers to performing exercise with light or medium loads while consuming oxygen. The anaerobic exercise refers to performing exercise with high loads without consuming oxygen. The exercise system 1 includes a load applying apparatus 10 and an ion delivery apparatus 20. The exercise system 1 of the present embodiment is disposed in a room 30.

The load applying apparatus 10 applies a load to the body of the user H. Specifically, the load applying apparatus 10 applies, to the body of the user H, a load that resists the movement of the body of the user H. The load refers to a force that opposes the movement of the user H. For example, when the user H runs, a force in the direction opposite to the direction in which the user H kicks the ground is applied to the user H. For example, when the user H moves his/her arm down, the load applying apparatus 10 applies, to the user H, a force in the direction opposite to the direction to lower the arm. The load applying apparatus 10 corresponds to an example of a "load applying unit."

The load applying apparatus 10 is, for example, an exercise machine. Examples of exercise machines include a tread mill, an exercise bike, a stepper machine, a weight machine, and an ergometer. The ergometer may be a rowing ergometer. The rowing ergometer is an exercise apparatus for performing body movements on or under water on land.

As illustrated in FIGS. 1 and 2, the user H is located on the load applying apparatus 10. Specifically, the user H is located in an exercise space 101 of the load applying apparatus 10. The user H exercises in the exercise space 101. The load applying apparatus 10 illustrated in FIGS. 1 and 2 is a tread mill. For example, the user H runs in the exercise space 101 of the tread mill. The user H runs in the exercise space 101, thus rotating the endless belt of the tread mill. The load applying apparatus 10 generates a force that opposes the opposite direction to the rotation direction of the endless belt. That is, a load that opposes the movement of the body of the user H can be applied to the body of the user H.

The ion delivery apparatus 20 is disposed in the room and delivers ions. The ion delivery apparatus 20 emits ions to the room 30. The ions are emitted from the ion delivery apparatus 20 to the room 30, filling the room 30 with the ions. That is, the concentration of ions in the room 30 is increased. When the user H enters the room 30 with an increased concentration of ions, the psychological state with regard to exercise is changed before exercising. Accordingly, the user H takes on a psychological state in which the user H can exercise in a positive mood before exercising. As a result, the effects of the exercise performed by the user H can be improved. The ion delivery apparatus 20 corresponds to an example of an "ion delivery unit." The ion delivery apparatus 20 is, for example, an air purifier with an ion generation function. The ion delivery apparatus 20 may be, for example, an air conditioner with an ion generation function or other air conditioning apparatuses with an ion generation function.

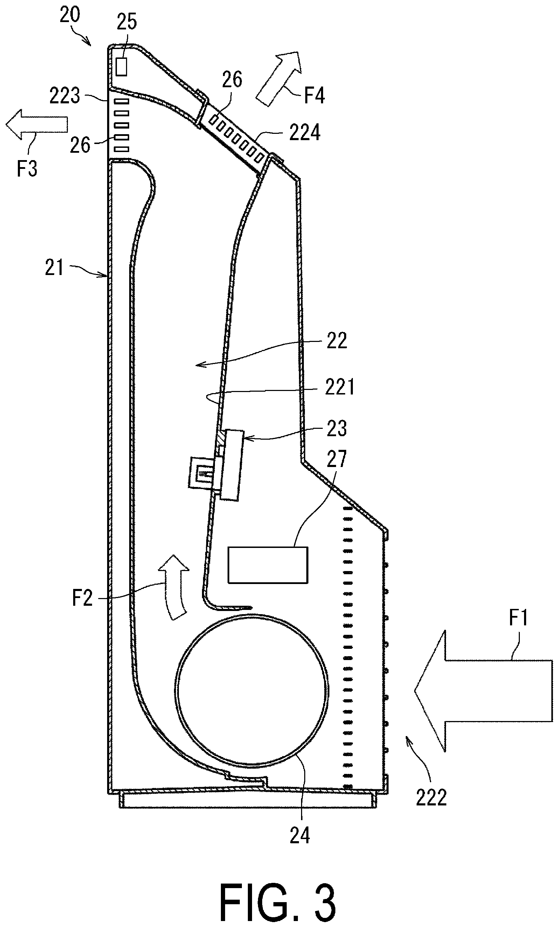

Next, with reference to FIG. 3, the ion delivery apparatus 20 will be described in detail. FIG. 3 is a diagram illustrating the ion delivery apparatus 20 according to the exercise system 1 of the present embodiment.

The ion delivery apparatus 20 includes a housing 21, a duct 22, an ion generation unit 23, an air sending unit 24, a detection unit 25, and louvers 26, and an adjustment unit 27.

The housing 21 houses the duct 22, the ion generation unit 23, the air sending unit 24, the detection unit 25, the louvers 26, and the adjustment unit 27. The housing 21 is the outer shell of the ion delivery apparatus 20.

The duct 22 guides air. The duct 22 includes a body portion 221, an inlet port 222, a first discharge port 223, and a second discharge port 224.

The inlet port 222 allows air to flow into the body portion 221. Accordingly, the air flows into the body portion 221 via the inlet port 222 from a first direction F1 side. The first direction F1 represents a direction from the outer side to the inner side of the body portion 221.

The body portion 221 is an air flow path. The body portion 221 guides the air flowing from the first direction F1 to a second direction F2. The second direction F2 refers to a direction from the inlet port 222 toward the first discharge port 223 or a direction from the inlet port 222 toward a second discharge port 224. Specifically, the body portion 221 guides the air flowing from the inlet port 222 to the first discharge port 223 and the second discharge port 224.

The first discharge port 223 discharges the air flowing into the body portion 221 to the atmosphere. Specifically, the first discharge port 223 discharges air in a third direction F3. The third direction F3 is a direction different from the direction in which the inlet port 222 is disposed. The third direction F3 is, for example, a direction from the second discharge port 224 toward the first discharge port 223.

The second discharge port 224 discharges the air flowing into the body portion 221 to the atmosphere. Specifically, the second discharge port 224 discharges air in a fourth direction F4. The fourth direction F4 discharges air in a direction different from the third direction F3. For example, the fourth direction F4 is a direction different from the direction in which the inlet port 222 is disposed. The fourth direction F4 is, for example, a direction from the first discharge port 223 toward the second discharge port 224.

The ion generation unit 23 generates ions. The ion generation unit 23 includes a pair of discharge electrodes. Each of the pair of discharge electrodes is a needle-like or brush-like electrode. The pair of discharge electrodes is each formed of, for example, a metal having electrical conductivity.

The pair of discharge electrodes discharge. Specifically, the pair of discharge electrodes to which a high voltage is applied discharge and generate active species. The active species contain ions. For example, the pair of discharge electrodes to which a high voltage is applied generate a corona discharge between the pair of discharge electrodes. Then, one of the pair of discharge electrodes emits positive ions via discharge. The positive ions are cluster ions (H⁺(H₂O)ₘ, where m is any integer of zero or greater) with a plurality of water molecules clustered around a hydrogen ion (H⁺). Also, the other of the pair of discharge electrodes emits negative ions via discharge. The negative ions are cluster ions (O₂⁻(H₂O)ₙ, where n is any integer of zero or greater) with a plurality of water molecules clustered around an oxygen ion (O₂⁻).

The emitted positive ions and negative ions each take in, for example, molds and general bacteria in the air and undergo a chemical reaction on the surfaces of the molds and the general bacteria. The active species hydroxyl radical (•OH) is produced by the chemical reaction. Then, the molds and general bacteria are removed by the hydroxyl radical (•OH).

The ion generation unit 23 is disposed on the duct 22. Specifically, the ion generation unit 23 is disposed at a position between the inlet port 222 and the first discharge port 223. The ion generation unit 23 may also be disposed at a position between the inlet port 222 and the second discharge port 224.

The air sending unit 24 generates wind. The air sending unit 24 is, for example, a fan. The fan is, for example, a sirocco fan. Specifically, the air sending unit 24 generates wind in the direction from the inlet port 222 toward the first discharge port 223. Also, the air sending unit 24 generates wind in the direction from the inlet port 222 toward the second discharge port 224. The air sending unit 24 is disposed in the inlet port 222.

The wind generated by the air sending unit 24 contains ions generated by the ion generation unit 23. That is, the wind containing the ions is discharged from the first discharge port 223 and the second discharge port 224. Then, the ions are emitted to the room 30. The ions are emitted from the ion delivery apparatus 20 to the room 30, filling the room 30 with the ions.

The detection unit 25 detects the user H. Specifically, the detection unit 25 detects a portion where the skin of the user H is exposed. The portion where the skin is exposed may be, for example, the face of the user H.

The louvers 26 are rotatably attached to the housing 21. Specifically, in FIG. 3, the louvers 26 are attached to the housing 21 in a manner allowing for rotation about a rotation axis (not illustrated). The rotation axis of the louvers 26 is, for example, substantially parallel to the horizontal direction. Also, the louvers 26 are detachably attached to the housing 21. Also, the louvers 26 are attached to the housing 21 so as to face the first discharge port 223. Also, the louvers 26 are attached to the housing 21 so as to face the second discharge port 224.

For example, in the first discharge port 223, the louvers 26 guide the wind passing through the interior of the duct 22 in a direction corresponding to the rotation angle of the louvers 26. That is, the direction of the wind to be discharged from the first discharge port 223 is a direction corresponding to the rotation angle of the louvers 26. Also, for example, in the second discharge port 224, the wind passing through the interior of the duct 22 is guided in a direction corresponding to the rotation angle of the louvers 26. That is, the direction of the wind to be discharged from the second discharge port 224 is a direction corresponding to the rotation angle of the louvers 26.

The adjustment unit 27 adjusts the rotation angle of the louvers 26. Specifically, the adjustment unit 27 adjusts the rotation angle of the louvers 26 attached to the first discharge port 223. Also, the adjustment unit 27 adjusts the rotation angle of the louvers 26 attached to the second discharge port 224. Thus, the angle of the louvers 26 can be adjusted to discharge wind containing ions. As a result, the wind containing the ions can be discharged in the desired direction of the user H.

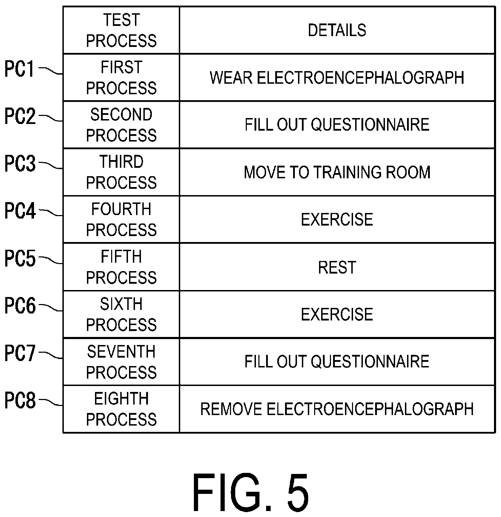

Further, with reference to FIGS. 2 and 3, the ion delivery apparatus 20 will be described in detail. The ion delivery apparatus 20 delivers ions toward the load applying apparatus 10. That is, the ion delivery apparatus 20 can deliver ions to the user H using the load applying apparatus 10. The ions delivered in the direction of the user H reach the user H. Thus, the ions that reach the user H change the psychological state (at least one of activity level, arousal level, comfort level, fatigue and concentration) of the user H with regard to exercise. As a result, the effects of the exercise performed by the user H can be objectively or subjectively improved. For example, the psychological state of the user H with regard to exercise is changed, and the user H can exercise in a positive mood. That is, the ions affect the psychological state of the user H with regard to exercise, and the user H can exercise in a psychological state suited to exercise. As a result, the effects of the exercise performed by the user H can be improved. Also, the user H exercises in the room 30 filled with the delivered ions. In other words, the ion delivery apparatus 20 emits ions in the space of the room 30 while also emitting ions in the direction of the user H. Thus, ions can be further delivered to the user H exercising in the room 30 filled with ions. As a result, the ions can directly and indirectly affect the user H, allowing the effects of the exercise performed by the user H to be improved. Note that the effects of the exercise can also be improved by exercising in an environment with air purified by the ions delivered from the ion delivery apparatus 20. The psychological states (activity level, arousal level, comfort level, and fatigue) of the user H with regard to exercise will be described below using examples with reference to FIGS. 5 to 13.

In addition, the ion delivery apparatus 20 according to the present embodiment delivers ions in the direction of the user H exercising using the load applying apparatus 10. The ions affect the psychological state of the user H with regard to exercise. Thus, the user H can exercise in a psychological state suited to exercise. For example, the user H can concentrate more on the exercise. As a result, the effects of the exercise can be further improved.

In addition, the ion delivery apparatus 20 according to the present embodiment delivers ions to a specific portion H1 of the user H. The specific portion H1 is a portion where the skin of the user H is exposed. Thus, ions can be delivered to the portion where the skin of the user H is exposed. The ions delivered to the skin can act on the body. Also, the user H may breathe in the ions delivered from the ion delivery apparatus 20 or breath in air purified by the ions. As a result, the ions can directly affect the user H. Also, the user H exercises in the room 30 filled with the delivered ions. Thus, the ions filling the room 30 can indirectly affect the user H. As a result, the ions can directly and indirectly affect the user H, allowing the effects of the exercise performed by the user H to be further improved.

For example, in a case where ions are delivered to clothing worn by the user H, the ions may disappear at the location of the clothing. That is, the number of ions that reach the user H is decreased. However, the ion delivery apparatus 20 of the present embodiment can deliver ions to a portion where the skin of the user H is exposed. This can reduce a decrease in the number of ions that reach the specific portion H1 of the user H. As a result, the ions delivered from the ion delivery apparatus 20 can act more directly on the user H.

Also, the portion where the skin of the user H is exposed is, for example, the "face". By delivering ions to the face, the user H may breathe in the air containing the ions. This results in the ions more directly affecting the user H which in turn facilitates the user H having a psychological state suited to the exercise.

For example, in a case where ions are delivered to the "face" of the user H, the adjustment unit 27 adjusts the rotation angle of the louvers 26 on the basis of a detection result of the detection unit 25. Then, the ion generation unit 23 generates ions. The air sending unit 24 further generates wind. Here, the wind generated by the air sending unit 24 includes ions generated by the ion generation unit 23. The wind containing the ions are released from the first discharge port 223 or the second discharge port 224. Then, the wind is guided by the louver 26 with the rotation angle adjusted by the adjustment unit 27. That is, the ions emitted from the first discharge port 223 are guided in the third direction F3 by the louvers 26 adjusted by the adjustment unit 27. For example, as illustrated in FIG. 2, the ions emitted in the third direction F3 reach the user H located in the exercise space 101. This results in the wind containing ions being delivered to the "face" of the user H. Note that the specific portion H1 may be a hand or a foot. For example, in a case where the hand is exposed, the wind containing ions may be delivered to the hand. For example, in a case where the foot is exposed, the wind containing ions may be delivered to the foot.

In addition, the ion delivery apparatus 20 may deliver the ions to the room 30 before the user H uses the load applying apparatus 10. Specifically, the ion delivery apparatus 20 may deliver the ions to the load applying apparatus 10 before the user H uses the load applying apparatus 10. Thus, before the user H who entered the room 30 exercises, the psychological state of the user H can be put in a psychological state suited to exercise. As a result, the effects of the exercise can be further improved.

In addition, the ion delivery apparatus 20 may deliver the ions to the room 30 after the user H uses the load applying apparatus 10. Specifically, the ion delivery apparatus 20 may deliver ions in the direction of the user H after the user H uses the load applying apparatus 10. Thus, the user H can be put in a psychological state suited to exercise until the user H exits the room 30. As a result, the effects of the exercise performed by the user H can be improved even when exercise is started again.

Next, the ion delivery apparatus 20 according to the present embodiment will be described in more detail with reference to FIGS. 1 to 3. As illustrated in FIGS. 1 and 2, the exercise system 1 may be provided with a plurality of ion delivery apparatuses 20.

As illustrated in FIGS. 1 and 2, the plurality of ion delivery apparatuses 20 are disposed at different positions from one another. That is, the ions can be delivered from different positions to the load applying apparatus 10 or the user H. This makes it easy to increase the concentration of ions around the load applying apparatus 10 or the user H. As a result, even when the position of the user H moves during exercise, the ions can affect the user H and can put the user H in a psychological state suited to exercise.

The plurality of ion delivery apparatuses 20 includes a first ion delivery apparatus 200A, a second ion delivery apparatus 200B, a third ion delivery apparatus 200C, a fourth ion delivery apparatus 200D, a fifth ion delivery apparatus 200E, and a sixth ion delivery apparatus 200F. The first ion delivery apparatus 200A to the sixth ion delivery apparatus 200F are air purifiers with an ion generation function. Each one of the first ion delivery apparatus 200A to the sixth ion delivery apparatus 200F may be, for example, an air conditioner with an ion generation function or other air conditioning apparatuses with an ion generation function. The first ion delivery apparatus 200A to the sixth ion delivery apparatus 200F have the same configuration as the ion delivery apparatus 20 described with reference to FIG. 3. Accordingly, detailed description of the first ion delivery apparatus 200A to the sixth ion delivery apparatus 200F is omitted.

For example, when the first ion delivery apparatus 200A delivers ions to the load applying apparatus 10 or the user H, the second ion delivery apparatus 200B to the sixth ion delivery apparatus 200F also deliver ions to the load applying apparatus 10 or the user H. For example, as illustrated in FIG. 2, the second ion delivery apparatus 200B to the sixth ion delivery apparatus 200F deliver ions from a position different from the first ion delivery apparatus 200A toward the load applying apparatus 10 or the user H. That is, the first ion delivery apparatus 200A to the sixth ion delivery apparatus 200F deliver ions toward the load applying apparatus 10 or the user H. Thus, the concentration of ions around the load applying apparatus 10 or the user H is increased. This facilitates the user H having a psychological state suited to the exercise.

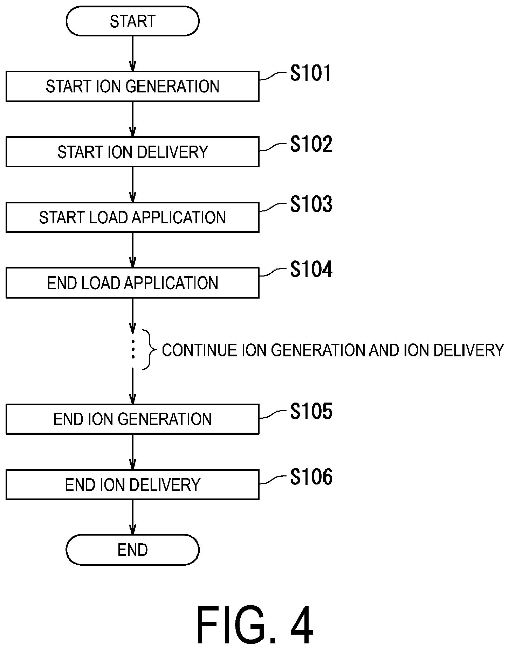

Next, with reference to FIG. 4, the processing executed by the exercise system 1 according to the present embodiment will be described. FIG. 4 is a diagram illustrating the processing executed by the exercise system 1 according to the present embodiment. The processing illustrated in FIG. 4 includes step S101 to step S106.

In step S101, the ion generation unit 23 of the ion delivery apparatus 20 starts ion generation. The processing then proceeds to step S102.

In step S102, the air sending unit 24 of the ion delivery apparatus 20 starts wind generation. The wind generated by the air sending unit 24 includes ions generated by the ion generation unit 23. The processing then proceeds to step S103.

In step S103, the load applying apparatus 10 starts load application to the body of the user H. The processing then proceeds to step S104.

In step S104, the load applying apparatus 10 ends the load application to the body of the user H. The processing then proceeds to step S105.

In step S105, the ion generation unit 23 of the ion delivery apparatus 20 ends the ion generation. The processing then proceeds to step S106.

In step S106, the air sending unit 24 of the ion delivery apparatus 20 ends the wind generation. The processing then ends.

Note that in the processing executed by the exercise system 1 according to the present embodiment, step S103 may be executed at the same time as step S101 and step S102. Further, in the processing executed by the exercise system 1 according to the present embodiment, step S101 and step S102 may be executed after the end of step S104. In a case where step S101 and step S102 are executed after the end of step S104, step S101 and step S102 correspond to an example of "delivering executed after applying." Further, in the processing executed by the exercise system 1 according to the present embodiment, step S101 and step S102 may be executed while step S103 is being executed.

In the processing executed by the exercise system 1, step S103 corresponds to an example of "applying a load to the body of the user H." In the processing executed by the exercise system 1, step S101 and step S102 correspond to an example of "delivering ions." Also, step S101 and step S102 may correspond to an example of "delivering executed before applying."

### Examples

Referring now to FIGS. 5 to 13, an example using an exercise method according to the present invention will be described together with a comparative example. The present invention will be described in detail on the basis of the example, but the present invention is not limited to the following example.

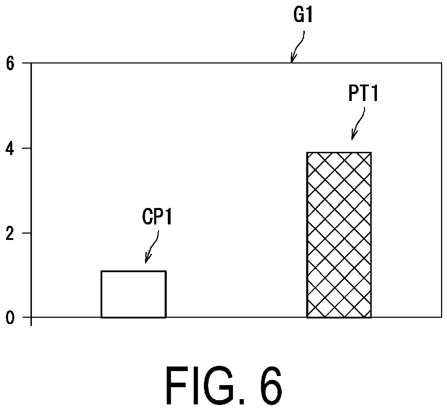

First, with reference to FIG. 5, an experimental process using an exercise method according to the present invention will be described. FIG. 5 is a diagram showing an experimental process using an exercise method according to the present invention. The experimental process includes a first process PC1 to an eighth process PC8. Also, ten test subject exercise according to the experimental processes listed in FIG. 5. Note that in FIGS. 6 to 13, the experimental results of the ten test subjects that exercised according to the experimental processes listed in FIG. 5.

The first process PC1 refers to a process in which the test subjects wear an electroencephalograph. A simple electroencephalograph is used as the electroencephalograph. The electrodes of the electroencephalograph are attached to the ear and forehead of the test subject. The electroencephalograph acquires α waves and β waves every second. The electroencephalogram acquired from the simple electroencephalograph is converted to a value indicating the psychological state of the user H by a sensitivity module logger (available from Little Software Co., Ltd.).

The sensitivity module logger Fourier converts the α waves and the β waves, for example. The sensitivity module logger extracts the feature points for each frequency and outputs a numerical values obtained by combining the extracted feature points. Further, the sensitivity module logger calculates an indicator indicating the psychological state (emotion) of each test subject on the basis of the numerical value. Note that in the first process PC1, immediately after the test subjects put on the electroencephalograph device, the electroencephalograph measures the brain waves of the test subjects. The test subjects start the second process PC2 two minutes after putting on the electroencephalograph.

The second process PC2 refers to a process in which the test subjects answer a questionnaire. The questionnaire of the second process PC2 is filled out outside of the training room. The questionnaire is a two-dimensional mood scale-short term (TDMS-ST) questionnaire. The two-dimensional mood scale-short term (TDMS-ST) questionnaire can measure changes in the mood (psychological state) of the user H.

The third process PC3 refers to a process in which the test subjects move to a first training room or a second training room. The ion delivery apparatuses 20 are disposed in the room 30 of the first training room. In the first training room of the third process PC3, ions are delivered to the user H. The exercise space 101 of the room 30 of the first training room is given an ion concentration of 100000 ions per 1 cm³. The ion delivery apparatuses 20 are disposed in the room of the second training room. The ion delivery apparatuses 20 in the second training room are controlled so that no ions are delivered. In the second training room of the third process PC3, a wind containing no ions is delivered to the user H. Also, the temperature of the first training room and the second training room is 24 degrees, and the humidity of the first training room and the second training room is 60% ± 2.0. In addition, the test subjects in the first training room do not know that the wind being delivered contains ions from the ion delivery apparatus 20. The test subjects in the second training room do not know that the wind being delivered contains no ions from the ion delivery apparatus 20.

The fourth process PC4 refers to a process in which the test subjects exercise. The exercise performed by the test subjects in the fourth process PC4 is performed on the load applying apparatus 10, as illustrated in FIG. 2. Specifically, in the exercise in the fourth process PC4, a swim ergometer (available from Concept 2) is used. The test subjects performed eight repetitions of 20 seconds of exercise using the swim ergometer (available from Concept 2). The test subjects rested for ten seconds between each 20 second repetition of exercise.

The fifth process PC5 refers to a process in which the test subjects rest. In the fifth process PC5, the test subjects lay face-up on a mat for ten minutes with their eyes closed.

The sixth process PC6 refers to a process in which the test subjects exercise. In the sixth process PC6, as in the fourth process PC4, a swim ergometer (available from Concept 2) is used. The exercise of the sixth process PC6 includes only one repetition of 20 seconds of exercise.

The seventh process PC7 refers to a process in which the test subjects answer a questionnaire. For the questionnaire of the seventh process PC7, a two-dimensional mood scale-short term (TDMS-ST) questionnaire and a rate of perceived exertion (RPE) questionnaire was used. With a rate of perceived exertion (RPE) questionnaire, the person exercising indicates via a numerical value how "hard" the exercise is. In the rate of perceived exertion questionnaire, the test subjects subjectively select numerical values ranging from 1 to 20. Higher numerical values indicate a higher rate of exertion. The test subjects answer the questionnaire in regard to the exercise of the fourth process PC4 and in regard to the exercise of the sixth process PC6.

The eighth process PC8 refers to a process in which the test subjects remove the electroencephalograph. The electroencephalograph does not measure the brain waves for the minute before it is removed.

### Examples and Comparative Examples

Referring to FIGS. 6 to 13, an example of the present invention and a comparative example will be described. The example shows results of experiments performed in the first training room. The comparative example shows results of experiments performed in the second training room.

FIG. 6 is a graph showing the average values of the amount of change in the activity level of the user H in the example of the present invention and the comparative example. FIG. 6 shows a graph G1. The graph G1 shows an experimental result PT1 and an experimental result CP1. The experimental result PT1 shows the average value of the amount of change in the activity level calculated on the basis of the questionnaire results of ten test subjects who exercised in the room 30 in which the plurality of ion delivery apparatuses 20 were disposed. The experimental result CP1 shows the average value of the amount of change in the activity level calculated on the basis of the questionnaire results of ten test subjects who exercised in a room in which the plurality of ion delivery apparatuses 20 were not disposed.

The experimental result PT1 and the experimental result CP1 shown in FIG. 6 each indicate the average value of the amount of change in the activity level measured by performing a two-dimensional mood scale-short term (TDMS-ST) questionnaire. The activity level is an indicator indicating how much energy the user H feels is in their body. Specifically, the activity level indicates a standard for the psychological state with a positive feeling of exhilaration and a negative feeling of heaviness on either end. For example, when the numerical value indicating the activity level is high, the user H is a state in which they have lots of energy. When the numerical value indicating the activity level is low, the user H is a state in which they feel lethargic and are without energy.

As shown in FIG. 6, the experimental result PT1 of the example was a result significantly larger than the experimental result CP1 of the comparative example. Specifically, the average value of the amount of change in the activity level of the example shown in FIG. 6 is "about 4." On the other hand, the average value of the amount of change in the activity level of the comparative example is "about 1." As shown in FIG. 6, the amount of change in the activity level of the example is larger than the amount of change in the activity level of the comparative example. Larger amounts of change in the activity level indicate that the user H is exercising in an energetic state. That is, it is possible to infer that the user H of the example was exercising with lots of energy compared to the user H of the comparative example. Thus, larger amounts of change in the activity level indicate a psychological state suited to exercise. Thus, it is possible to infer that the user H that exercised under the conditions of the example exercised with a psychological state more suited to exercise than the user H that exercised under the conditions of the comparative example.

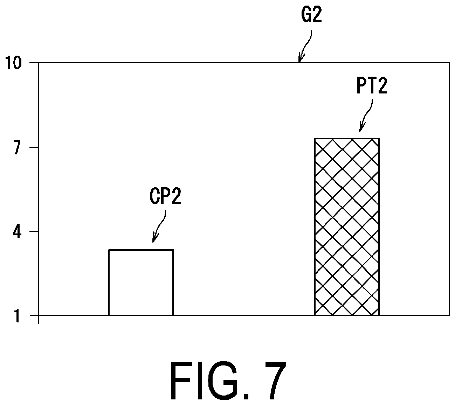

Referring to FIG. 7, an example of the present invention and a comparative example will be described. FIG. 7 is a graph showing the average values of the amount of change in the arousal level in the example of the present invention and the comparative example. FIG. 7 shows a graph G2. The graph G2 shows an experimental result PT2 and an experimental result CP2. The experimental result PT2 shows the average value of the amount of change in the arousal level calculated on the basis of the questionnaire results of ten test subjects who exercised in the room 30 in which the plurality of ion delivery apparatuses 20 were disposed. The experimental result CP2 shows the average value of the amount of change in the arousal level calculated on the basis of the questionnaire results of ten test subjects who exercised in a room in which the plurality of ion delivery apparatuses 20 were not disposed.

The experimental result PT2 and the experimental result CP2 shown in FIG. 7 each indicate the average value of the amount of change in the arousal level measured by performing a two-dimensional mood scale-short term (TDMS-ST) questionnaire. The arousal level is an indicator indicating the arousal level of the user H.

As shown in FIG. 7, the experimental result PT2 of the example was a result significantly higher than the experimental result CP2 of the comparative example. Specifically, the average value of the amount of change in the arousal level of Example 1 shown in FIG. 7 is "about 7." On the other hand, the average value of the amount of change in the arousal level of Comparative Example 1 is "about 3." As shown in FIG. 7, the amount of change in the arousal level of Example 1 is larger than the amount of change in the arousal level of Comparative Example 1. Larger amounts of change in the arousal level indicate that the user H is exercising in an aroused state. Also, larger amounts of change in the arousal level indicate that the user H has a psychological state suited to exercise. That is, it is possible to infer that the user H of Example 1 exercised, for example, in a psychological state of being exhilarated and invigorated compared to the user H of the comparative example. Thus, it is possible to infer that the user H that exercised under the conditions of Example 1 exercised with a psychological state more suited to exercise than the user H that exercised under the conditions of the comparative example.

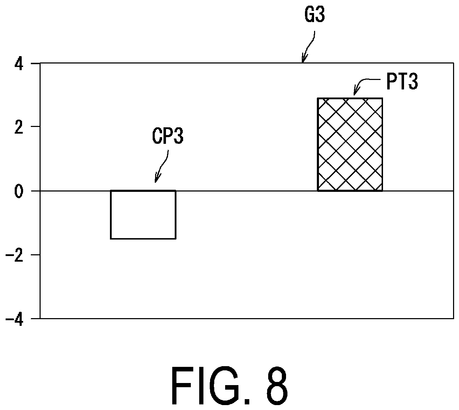

Referring to FIG. 8, Example 1 of the present invention and Comparative Example 1 will be described. FIG. 8 is a graph showing the average values of the amount of change in the comfort level in the example of the present invention and the comparative example. FIG. 8 shows a graph G3. The graph G3 shows an experimental result PT3 and an experimental result CP3. The experimental result PT3 shows the average value of the amount of change in the comfort level calculated on the basis of the questionnaire results of ten test subjects who exercised in the room 30 in which the plurality of ion delivery apparatuses 20 were disposed. The experimental result CP3 shows the average value of the amount of change in the comfort level calculated on the basis of the questionnaire results of ten test subjects who exercised in a room in which the plurality of ion delivery apparatuses 20 were not disposed.

The experimental result PT3 and the experimental result CP3 shown in FIG. 8 each indicate the average value of the amount of change in the comfort level measured by performing a two-dimensional mood scale-short term (TDMS-ST) questionnaire. The comfort level is an indicator indicating how comfortable the user H is feeling.

As shown in FIG. 8, the experimental result PT3 of the example was a result significantly larger than the experimental result CP3 of the comparative example. Specifically, the average value of the amount of change in the comfort level of the example shown in FIG. 8 is "about 3." On the other hand, the average value of the amount of change in the comfort level of the comparative example is "about -1.5." As shown in FIG. 8, the amount of change in the comfort level of the example is larger than the amount of change in the comfort level of the comparative example. Larger amounts of change in the comfort level indicate that the user H is exercising while feeling comfortable. Also, larger amounts of change in the comfort level indicate that the user H has a psychological state suited to exercise. Thus, it is possible to infer that the user H that exercised under the conditions of the example exercised with a psychological state more suited to exercise than the user H that exercised under the conditions of the comparative example.

As described in the examples, with regard to the activity level, arousal level, and the comfort level, the experimental results PT1 to PT3 when exercise is performed using the ion delivery apparatuses 20 are significantly larger in terms of the average value of the amount of change measured by a two-dimensional mood scale-short term (TDMS-ST) questionnaire than the experimental results CP1 to CP3 when exercise is performed without using the ion delivery apparatuses 20. That is, it can be inferred that the effects of exercise can be improved by exercising while ions are delivered by the ion delivery apparatuses 20.

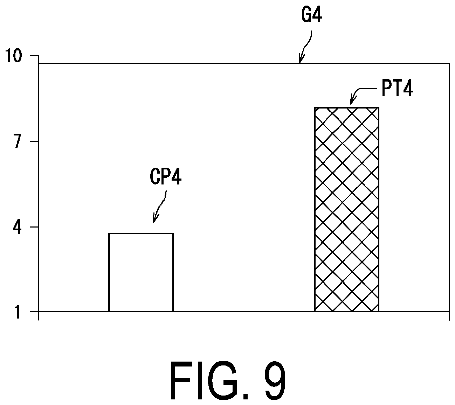

Referring to FIG. 9, an example of the present invention and a comparative example will be described. FIG. 9 is a graph showing the average values of the amount of change in the activity level in the example of the present invention and the comparative example. FIG. 9 shows a graph G4. The graph G4 shows an experimental result PT4 and an experimental result CP4. The experimental result PT4 shows the average value of the amount of change in the activity level calculated on the basis of the brain waves acquired from ten test subjects who exercised in the room 30 in which the plurality of ion delivery apparatuses 20 were disposed. The experimental result CP4 shows the average value of the amount of change in the activity level calculated on the basis of the brain waves acquired from ten test subjects who exercised in a room in which the plurality of ion delivery apparatuses 20 were not disposed.

FIG. 9 shows the activity level measured from the brain waves of the test subjects using an electroencephalograph. The activity level is output using a sensitivity module logger (available from Little Software Co., Ltd.) on the basis of the value output from the electroencephalograph. The activity level is a value calculated from a frequency analysis of the brain waves. The calculated value is calculated from, for example, a value indicating an LF (Low Frequency) component and a value indicating a HF (Hi Frequency) component using a sensitivity module logger (available from Little Software Co., Ltd.).

As shown in FIG. 9, the experimental result PT4 of Example 1 was a result significantly higher than the experimental result CP4 of the comparative example. Specifically, the average value of the amount of change in the activity level of Example 1 shown in FIG. 9 is "about 8." On the other hand, the average value of the amount of change in the activity level of the comparative example is "about 4." As shown in FIG. 9, the amount of change in the activity level of Example 1 is larger than the amount of change in the activity level of the comparative example. Larger amounts of change in the activity level indicate that the user H is exercising with their brain in an energetic state. Thus, it is possible to infer that the user H that exercised under the conditions of Example 1 exercised with a psychological state more suited to exercise than the user H that exercised under the conditions of the comparative example.

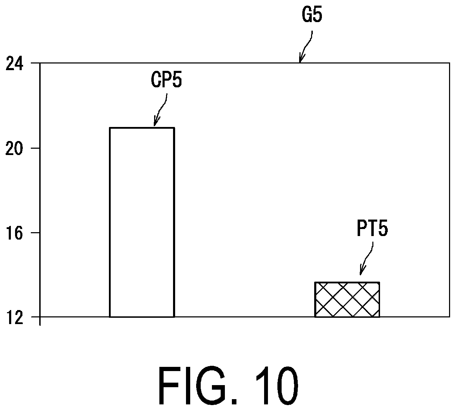

Referring to FIG. 10, Example 1 of the present invention and a comparative example will be described. FIG. 10 is a graph showing the average values of the amount of change in the fatigue level in the example of the present invention and the comparative example. FIG. 10 shows a graph G5. The graph G5 shows an experimental result PT5 and an experimental result CP5. The experimental result PT5 shows the average value of the amount of change in the fatigue level calculated on the basis of the brain waves acquired from ten test subjects who exercised in the room 30 in which the plurality of ion delivery apparatuses 20 were disposed. The experimental result CP5 shows the average value of the amount of change in the fatigue level calculated on the basis of the brain waves acquired from ten test subjects who exercised in a room in which the plurality of ion delivery apparatuses 20 were not disposed.

FIG. 10 shows the fatigue measured from the brain waves of the test subjects using an electroencephalograph. The fatigue level is output using a sensitivity module logger (available from Little Software Co., Ltd.) on the basis of the value output from the electroencephalograph. The fatigue level is a value calculated from a frequency analysis of the brain waves. The fatigue level is an indicator indicating the amount of fatigue the user H has.

As shown in FIG. 10, the experimental result CP5 of the comparative example tends to be larger than the experimental result PT5 of Example 1. Specifically, the amount of change in fatigue in Example 1 shown in FIG. 10 is "about 14." On the other hand, the average value of the amount of change in fatigue of the comparative example is "about 21." As shown in FIG. 10, the amount of change in fatigue of Example 1 is less than the amount of change in fatigue of the comparative example. Less amounts of change in fatigue indicate that the user H is exercising with an unfatigued brain. Thus, it is possible to infer that the user H that exercised under the conditions of Example 1 exercised with a psychological state more suited to exercise than the user H that exercised under the conditions of the comparative example.

As described with reference to FIG. 9, with regard to the activity level, the experimental result PT4 when exercise is performed using the ion delivery apparatuses 20 is significantly higher in terms of the amount of change in the psychological state measured using brain waves than the experimental result CP4 when exercise is performed without using the ion delivery apparatuses 20. Also, as described with reference to FIG. 10, with regard to the fatigue level, the experimental result PT5 when exercise is performed using the ion delivery apparatuses 20 tends to be less in terms of the amount of change in the psychological state measured using brain waves than the experimental result CP5 when exercise is performed without using the ion delivery apparatuses 20. That is, it can be inferred that the effects of exercise can be improved by exercising while ions are delivered by the ion delivery apparatuses 20.

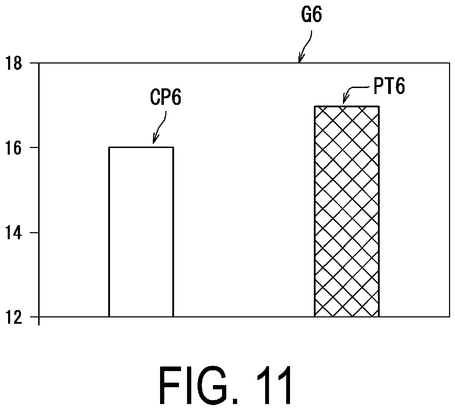

Referring to FIG. 11, Example 1 of the present invention and a comparative example will be described. FIG. 11 is a graph showing the average values of the rate of perceived exertion in the example of the present invention and the comparative example. FIG. 11 shows a graph G6. The graph G6 shows an experimental result PT6 and an experimental result CT6. The experimental result PT6 shows the average value of the rate of perceived exertion calculated on the basis of the questionnaire results of ten test subjects who exercised in the room 30 in which the plurality of ion delivery apparatuses 20 were disposed. The experimental result CT6 shows the average value of the rate of perceived exertion calculated on the basis of the questionnaire results of ten test subjects who exercised in a room in which the plurality of ion delivery apparatuses 20 were not disposed. The questionnaire is regarding the rate of perceived exertion (RPE).

As shown in FIG. 11, the experimental result PT6 of Example 1 tends to be higher than the experimental result CT6 of the comparative example. Specifically, the average value of the rate of perceived exertion (RPE) of Example 1 shown in FIG. 11 is "about 17." On the other hand, the average value of the rate of perceived exertion (RPE) of the comparative example is "about 16." As shown in FIG. 11, the average value of the rate of perceived exertion (RPE) of Example 1 is higher than the average value of the rate of perceived exertion (RPE) of the comparative example. The higher rate of perceived exertion (RPE) indicate that the user H is exercising with higher intensity. It is possible to infer that the user H of Example 1 was exercising with higher intensity than the user H of the comparative example. Thus, it is possible to infer that the user H that exercised under the conditions of Example 1 had improved effects of exercise over the user H that exercised under the conditions of the comparative example.

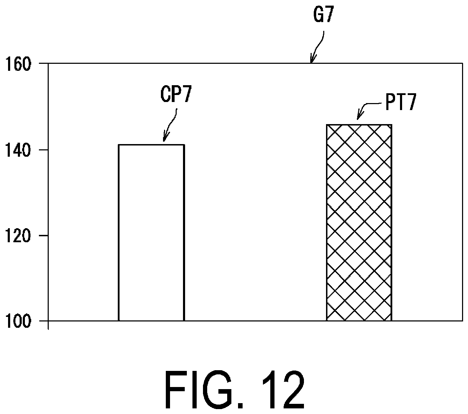

Referring to FIG. 12, an example of the present invention and a comparative example will be described. FIG. 12 is a graph showing the load amount of exercise in the example of the present invention and the comparative example. FIG. 12 shows a graph G7. The graph G7 shows an experimental result PT7 and an experimental result CP7. The experimental result PT7 shows the average value of the load amount of the exercise performed by ten test subjects in the room 30 in which the plurality of ion delivery apparatuses 20 were disposed. The experimental result CP7 shows the average value of the load amount of the exercise performed by ten test subjects in a room in which the plurality of ion delivery apparatuses 20 were not disposed. The load amount of the exercise shown in FIG. 12 indicates the average load applied to the user H during exercise.

As shown in FIG. 12, the experimental result PT7 of the example tends to be higher than the experimental result CP7 of the comparative example. Specifically, the average value of the load amount of the example shown in FIG. 12 is "about 146." On the other hand, the average value of the load amount of the comparative example is "about 141." As shown in FIG. 12, the average value of the rate of load amount of the example is higher than the average value of the load amount of the comparative example. That is, compared to exercising in the second training room, the exercise in the first training room is performed at a higher load amount. Thus, it is possible to infer that the user H of the example was exercising with a higher load amount than the user H of the comparative example. Thus, it is possible to infer that the user H that exercised under the conditions of the example had improved effects of exercise over the user H that exercised under the conditions of the comparative example.

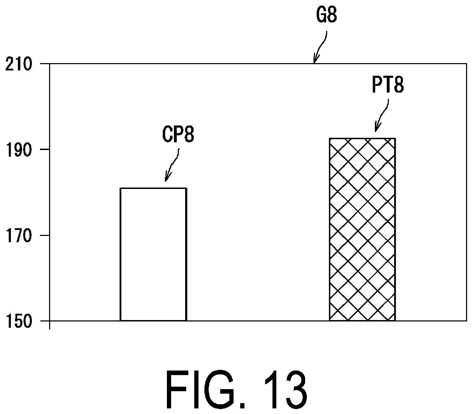

Referring to FIG. 12, an example of the present invention and a comparative example will be described. FIG. 13 is a graph showing the average values of the load amount of exercise of the sixth process PC6 in the example of the present invention and comparative example. FIG. 13 shows a graph G8. The graph G8 shows an experimental result PT8 and an experimental result CP8. The experimental result PT8 shows the average value of the load amount of the exercise of the sixth process PC6 performed by ten test subjects in the room 30 in which the plurality of ion delivery apparatuses 20 were disposed. The experimental result CP8 shows the average value of the load amount of the exercise of the sixth process PC6 performed by ten test subjects in a room in which the plurality of ion delivery apparatuses 20 were not disposed. The load amount of the exercise shown in FIG. 13 indicates the average load applied to the user H during exercise of the sixth process PC6.

As shown in FIG. 13, the experimental result PT8 of the example was a result significantly higher than the experimental result CT8 of the comparative example. Specifically, the average value of the load amount of the example shown in FIG. 13 is "about 193." On the other hand, the average value of the load amount of the comparative example is "about 181." As shown in FIG. 13, the average value of the rate of load amount of the example is higher than the average value of the load amount of the comparative example. That is, compared to exercising in the second training room, the exercise in the first training room is performed at a higher load amount. Thus, it is possible to infer that the user H of the example was exercising with a higher load amount than the user H of the comparative example. Thus, it is possible to infer that the user H that exercised under the conditions of the example had improved effects of exercise over the user H that exercised under the conditions of the comparative example.

As described using FIG. 11, the questionnaire result for the rate of perceived exertion (RPE) when exercise is performed using the ion delivery apparatuses 20 tends to be higher than the questionnaire result for the rate of perceived exertion (RPE) when exercise is performed without using the ion delivery apparatuses 20. Also, as described using FIG. 12, the load amount when exercise is performed using the ion delivery apparatuses 20 tends to be higher than the load amount when exercise is performed without using the ion delivery apparatuses 20. Also, as described using FIG. 13, the load amount when exercise of the sixth process PC6 is performed using the ion delivery apparatuses 20 is significantly higher than the load amount when exercise of the sixth process PC6 is performed without using the ion delivery apparatuses 20. That is, it can be inferred from the experimental results, PT6, PT7, and PT8 that the effects of exercise on the user H can be improved by exercising while ions are delivered by the ion delivery apparatuses 20.

An embodiment of the present invention has been described above with reference to the drawings. However, the present invention is not limited to the embodiment described above, and the present invention can be implemented in various modes without departing from the gist thereof. Further, various present inventions can be formed by appropriately combining a plurality of components disclosed in the embodiments described above. For example, several components may be deleted from all of the components described in the embodiments. Furthermore, the components across different embodiments may be appropriately combined. For ease of understanding, the drawings schematically illustrate each component as a main constituent, and the thickness, length, number, spacing, and the like of each component illustrated are different from the actual thickness, length, number, spacing for convenience of drawing preparation. Further, the speed, material, shape, dimensions, and the like of each component illustrated in the embodiments described above are one example and are not particularly limited, and various modifications can be made within a range that does not substantially deviate from the configuration of the present invention.
(1) In the exercise system 1 according to the present embodiment, the user H exercises in a training room in which the ion delivery apparatus 20 is disposed. However, no such limitation is intended. For example, the room 30 of the exercise system 1 may be a room in a moving body. In a case where the exercise system 1 is disposed in a moving body, the load applying apparatus 10 corresponds to an operation apparatus of the moving body.

The exercise system 1 of a moving body includes the load applying apparatus 10 and the ion delivery apparatus 20. The exercise system 1 is disposed in a room 30 of the moving body. The moving body is a car, a train, a watercraft, or an aircraft, for example. Additionally, a seat may be disposed in the room 30 of the moving body. The seat is sat on by the user H. The user H operates the moving body.

The load applying apparatus 10 of a first modified example applies a load to the body of the user H. For example, the load applying apparatus 10 applies a load to the user H by making the user H move the moving body. The load is, for example, the stress generated by moving the moving body.

The ion delivery apparatus 20 emits ions. The ion delivery apparatus 20 delivers ions toward the load applying apparatus 10. That is, the ion delivery apparatus 20 can deliver ions to the user H using the load applying apparatus 10. The ions delivered toward the user H reach the user H. Thus, the ions that reach the user H change the psychological state (at least one from among activity level, arousal level, comfort level, and fatigue) of the user H with regard to operating. For example, the psychological state of the user H with regard to operating is changed, and the user H can concentrate on operating. For example, the psychological state of the user H with regard to operating is changed, and the user H can operate with low stress. As a result, the user H can move the moving body such as the vehicle while concentrating and having low stress.

(2) The ion delivery apparatus 20 of the exercise system 1 according to the present embodiment may change the airflow on the basis of the heart rate of the user H. The heart beat of the user H can be obtained by a heart rate meter. The heart rate or ion delivery apparatus 20 is capable of wired or wireless communication with one another. That is, the heart beat obtained by the heart rate meter is transmitted to the ion delivery apparatus 20. The air sending unit 24 of the ion delivery apparatus 20 that acquires the heart beat of the user H changes the airflow on the basis of the acquired heart beat. In other words, the air sending unit 24 changes the airflow according to the acquired heart beat. Specifically, the air sending unit 24 increases the airflow as the acquired heart beat increases. Accordingly, the airflow of the air sending unit 24 can be changed in accordance with the heart rate of the user H. As a result, the airflow can be increased when the heart rate increases due to exercise. Note that the air sending unit 24 blows air as the ion generation unit 23 emits ions. Thus, ions are contained in the wind generated by the air sending unit 24. That is, in response to increasing the airflow, the number of ions delivered can also be increased. As a result, when the heart rate increases due to exercise, the wind containing the ions can be delivered to the user H.

Note that, in response to an increase in the heart rate, the adjustment unit 27 of the ion delivery apparatus 20 may adjust the rotation angle of the louvers 26. Thus, the angle of the louvers 26 can be adjusted to discharge wind containing ions. As a result, wind containing ions with an increase airflow can be discharged in the desired direction of the user H.

(3) The ion delivery apparatus 20 of the exercise system 1 according to the present embodiment may change the airflow on the basis of the training time. The training time refers to the amount of time during which the load applying apparatus 10 applies a load to the body of the user H. The ion delivery apparatus 20 acquires the training time from the load applying apparatus 10. Specifically, the load applying apparatus 10 and the ion delivery apparatus 20 are capable of wired or wireless communication with one another. That is, the training time measured by the load applying apparatus 10 is transmitted to the ion delivery apparatus 20. The air sending unit 24 of the ion delivery apparatus 20 that acquires the training time changes the airflow on the basis of the acquired training time. In other words, the air sending unit 24 changes the airflow according to the acquired training time. Specifically, the air sending unit 24 increases the airflow as the acquired training time increases. Accordingly, the airflow of the air sending unit 24 can be changed in accordance with the training time. As a result, the airflow can be increased when the heart rate increases due to exercise. Note that the air sending unit 24 blows air as the ion generation unit 23 emits ions. Thus, ions are contained in the wind generated by the air sending unit 24. That is, in response to increasing the airflow, the number of ions delivered can also be increased. As a result, when the training time increases, the wind containing the ions can be delivered to the user H.

Note that, in response to an increase in the training time, the adjustment unit 27 of the ion delivery apparatus 20 may adjust the rotation angle of the louvers 26. Thus, the angle of the louvers 26 can be adjusted to discharge wind containing ions. As a result, wind containing ions with an increase airflow can be discharged in the desired direction of the user H.

(4) The adjustment unit 27 of the ion delivery apparatus 20 of the exercise system 1 according to the present embodiment may change the wind direction on the basis of the type of load applying apparatus 10. For example, when exercising in a standing posture like when exercising on a treadmill, the adjustment unit 27 may adjust the rotation angle of the louvers 26 so that the wind containing the ions is delivered to the user H in the standing posture. For example, when exercising in a sitting posture like when exercising on a rowing ergometer, the adjustment unit 27 may adjust the rotation angle of the louvers 26 so that the wind containing the ions is delivered to the user H in the sitting posture. As a result, depending on the load applying apparatus 10 the user H uses to exercise, the rotation angle of the louvers 26 can be adjusted and the wind containing the ions can be delivered to the user H.

### Industrial Applicability

The present invention has applicability in the field of an exercise system, an exercise apparatus, and an exercise method.

### Reference Signs List

1 Exercise system
10 Load applying apparatus (load applying unit)
20 Ion delivery apparatus (ion delivery unit)
H User
S101 Step (applying step)
S102 Step (delivering step)
S103 Step (delivering step)

## Claims

1. An exercise system, comprising:
a load applying apparatus disposed in a room and configured to apply a load to a body of a user; and
an ion delivery apparatus disposed in the room and configured to deliver ions.

2. The exercise system according to claim 1,
wherein the ion delivery apparatus delivers ions toward a side of the load applying apparatus.

3. The exercise system according to claim 1 or 2,
wherein the ion delivery apparatus delivers the ions in a direction of the user exercising using the load applying apparatus.

4. The exercise system according to any one of claims 1 to 3,
wherein the ion delivery apparatus delivers the ions to a specific portion of the user, and
the specific portion refers to a portion where skin of the user is exposed.

5. The exercise system according to any one of claims 1 to 4, further comprising:
a plurality of the ion delivery apparatuses,
wherein the plurality of the ion delivery apparatuses is disposed at different positions and deliver ions from different directions.

6. An exercise apparatus, comprising:
a load applying unit disposed in a room and configured to apply a load to a body of a user; and
an ion delivery unit disposed in the room and configured to deliver ions.

7. The exercise apparatus according to claim 6, wherein the ion delivery unit delivers ions in a direction of the user.

8. An exercise method, comprising:
by a load applying unit disposed in a room, applying a load to a body of a user; and
by an ion delivery unit disposed in the room, delivering ions.

9. The exercise method according to claim 8,
wherein in the delivering, the ions are delivered in a direction of the user.

10. The exercise method according to claim 9,
wherein the delivering is executed before the applying.

11. The exercise method according to claim 9 or 10,
wherein the delivering is executed after the applying.
